# EUROPÄISCHE PATENTSCHRIFT

(11) **EP 2 498 739 B1**
(45) Veröffentlichungstag und Bekanntmachung des Hinweises auf die Patenterteilung: **08.04.2015**
(21) Anmeldenummer: 10795921.5
(22) Anmeldetag: 30.10.2010
(51) Int. Cl.: A61F 13/15, G01N 33/36, A61F 13/84

(54) **VERFAHREN ZUM BESTIMMEN DER FEUCHTIGKEIT BEI EINEM ABSORBIERENDEN HYGIENEARTIKEL UND VORRICHTUNG ZUR DURCHFÜHRUNG DES VERFAHRENS**
METHOD FOR DETERMINING THE MOISTURE CONTENT IN AN ABSORBENT SANITARY PRODUCT AND DEVICE FOR CARRYING OUT THE METHOD
PROCÉDÉ POUR DÉTERMINER L'HUMIDITÉ DANS UN ARTICLE HYGIÉNIQUE ABSORBANT ET DISPOSITIF POUR METTRE EN UVRE CE PROCÉDÉ

(30) Priorität: 12.11.2009 DE 102009054097
(43) Veröffentlichungstag der Anmeldung: 19.09.2012
(73) Patentinhaber: Paul Hartmann Aktiengesellschaft, 89522 Heidenheim (DE)
(72) Erfinder: PAZ, Rui, Miguel, 47800 Krefeld (DE); BECKERT, Susanne, 89073 Ulm (DE); KESSELMEIER, Rüdiger, 89542 Herbrechtingen (DE); SWEREV, Maximilian, 86169 Augsburg (DE); GAUSE, Enno, 89522 Heidenheim (DE); KRÜGER, Tim, 89073 Ulm (DE); BODMER, Magnus, 89231 Neu-Ulm (DE); GASSNER, Oliver, 50674 Köln (DE)
(74) Vertreter: DREISS Patentanwälte PartG mbB
(86) Internationale Anmeldenummer: PCT/EP2010/006639
(87) Internationale Veröffentlichungsnummer: WO 2011/057723

(56) Entgegenhaltungen:
- WO-A2-03/030771
- DE-A1-102006 053 405
- US-A1- 2004 043 369
- US-A1- 2007 048 709
- US-B1- 6 298 714

## Beschreibung

Die Erfindung befasst sich mit Prüfverfahren zum Beurteilen der Funktionsweise eines absorbierenden Hygieneartikels, insbesondere einer Windel, Inkontinenzvorlage oder -windel, Damenbinde oder dergleichen, wobei der Hygieneartikel beispielsweise im Hinblick auf die Flüssigkeitsaufnahme, die Flüssigkeitsverteilung, das Rücknässungsverhalten oder die Restfeuchtigkeit untersucht werden soll. Dies geschieht im Besonderen durch ein Verfahren zum Bestimmen der Feuchtigkeit, nachdem der Hygieneartikel zuvor unter vorzugsweise vorgegebenen Bedingungen mit Flüssigkeit beaufschlagt wurde.

Es sind im Stand der Technik zahlreiche Test- und Messverfahren zum Bestimmen und Beurteilen der Absorptionscharakteristik von absorbierenden Hygieneartikeln der hier in Rede stehenden Art bekannt. Beispielsweise beschreibt EP 0 887 055 A1 einen standardisierten Vorgang zum Einnässen eines Hygieneartikels unter Verwendung einer dem menschlichen Körper nachempfundenen Puppe, wobei bei der Windel ein Feuchtigkeitssensor angeordnet wird, der beobachtet wird.

Ferner offenbart diese Druckschrift einen im Labor durchzuführenden standardisierten Einnässungsvorgang bei einem Hygieneartikel im flächenhaft ausgebreiteten Zustand. Es wird auch ein Test zur Bestimmung des Rücknässungsverhaltens beschrieben, wobei Abschnitte eines Collagen-Filmmaterials auf die zuvor eingenässte Stelle unter Druck aufgebracht und deren Flüssigkeits- oder Feuchtigkeitsaufnahme bestimmt wird.

Ein ähnlicher Test ist beschrieben in der Prüfmethode UNE 153601-2 der spanischen Normbehörde AENOR. Auch hier wird ein flächenhaft ausgebreiteter Hygieneartikel in der Mitte der Produktlänge mit Flüssigkeit beaufschlagt, und es wird dort die Rücknässung in ein aufgelegtes und angedrücktes Filterpapier bestimmt.

Auch der Medizinische Dienst Deutschland hat ein Testverfahren in Teil 3 der Prüfvorschrift 1/93 MDS-HI bereitgestellt, wonach ebenfalls die Rücknässung nach Flüssigkeitseinleitung unter Druckbelastung und Bestimmung der Rücknässung unter Verwendung von Filterpapieren bestimmt wird, jedoch wird bei diesem Verfahren zunächst an mehreren repräsentativen Stellen aus einem Inkontinenzprodukt eine Probe genommen und diese mit synthetischem Urin beladen und vermessen. Das Verfahren arbeitet also nicht zerstörungsfrei und damit praxisfern.

Ein ähnliches Verfahren ist in der italienischen Prüfmethode NMC 93 Teilenr. 1 bis 3 zur Prüfung von Absorptionskapazität, Absorptionsgeschwindigkeit und Rückhaltevermögen von Inkontinenzprodukten beschrieben. Hierbei werden aus einem Inkontinenzprodukt 100 x 100 mm messende Proben aus einem vorderen, mittigen und einem hinteren Bereich entnommen. Die entnommenen Proben werden eingenässt und dann auf ein Ablaufgitter gestellt und in vorgegebener Weise belastet. Danach wird die Rücknässung als Differenz zwischen trockenem und nassem Probenstück ermittelt.

Durch die Zerstörung des absorbierenden Bereichs des Hygieneartikels und die vollständige Einnässung der Proben ist der Test für tatsächlich auftretende Tragesituationen des nicht zerstörten Hygieneartikels wenig aussagekräftig.

Bei den bekannten Verfahren wird der jeweils zu messende Parameter in praxisfernen Prüfsituationen bestimmt, die sich wesentlich von tatsächlich im Gebrauch eines Hygieneartikels auftretenden Tragesituationen unterscheiden. Die Ergebnisse der bekannten Testverfahren lassen daher keine zuverlässigen Rückschlüsse auf die Funktionsweise eines Hygieneartikels im tatsächlichen Gebrauch zu. Dies wurde mit der vorliegenden Erfindung erkannt.

Mit DE 10 2006 053 405 A1 wurde vorgeschlagen, einen Hygieneartikel im an einen Trägerdummy angelegten Zustand einzunässen und die Ausbreitung der Flüssigkeitsfront messtechnisch zu beobachten.

Der vorliegenden Erfindung lag die Aufgabe zugrunde, ein Verfahren der eingangs genannten und im Oberbegriff des Anspruchs 1 beschriebenen Art bereitzustellen, das es erlaubt, realistische Rückschlüsse auf das Betriebsverhalten und insbesondere die Flüssigkeitsaufnahme-, Flüssigkeitsverteilungs- oder Rücknässungscharakteristik eines Hygieneartikels zu ziehen.

Diese Aufgabe wird bei einem Verfahren der gattungsgemäßen Art erfindungsgemäß dadurch gelöst, dass über die flächenhafte Erstreckung des ausgebreiteten Hygieneartikels eine Mehrzahl zonierter Messareale definiert wird und dass in den jeweiligen Messarealen oder in Bezug auf die jeweiligen Messareale jeweils ein Messwert zum quantitativen Bestimmen der Feuchtigkeit in dem jeweiligen Messareal genommen wird.

Mit der vorliegenden Erfindung wird also vorgeschlagen, dass ein Hygieneartikel nach einer die Wirklichkeit möglichst gut simulierenden Einnässung an einer Vielzahl von Stellen betrachtet und messtechnisch erfasst werden sollte, um realistische Aussagen über das Betriebsverhalten des Hygieneartikels gewinnen zu können. Dadurch dass der ganze unzerstörte Hygieneartikel, vorzugsweise in seiner auch in der wirklichen Tragesituation zum Einsatz kommenden Konfiguration, eingenässt und dann an einer Vielzahl von Stellen, nämlich in den zonierten Messarealen, messtechnisch erfasst wird, lassen sich realistische Aussagen über das Betriebsverhalten des Hygieneartikels, und zwar detailliert für die jeweils betrachteten zonierten, d.h. an reproduzierbaren Stellen des Hygieneartikels liegenden Messareale, gewinnen. - Es sei auch darauf hingewiesen, dass im Sinne der vorliegenden Erfindung unter die Bezeichnung "Bestimmen der Feuchtigkeit bei einem Hygieneartikel" jegliche Messwertnahme zu subsummieren ist, die im Zusammenhang mit der in dem Hygieneartikel in den jeweiligen zonierten Messarealen aufgenommenen Flüssigkeitsmenge steht.

Dabei erweist es sich als besonders vorteilhaft, wenn die Messareale wenigstens 50 %, insbesondere wenigstens 60 %, insbesondere wenigstens 70 %, insbesondere wenigstens 80 % und weiter insbesondere wenigstens 90 % der flächenhaften Erstreckung des absorbierenden Bereichs des Hygieneartikels erfassen, wobei unter dem absorbierenden Bereich die zur dauerhaften Speicherung dienenden Schichten zu verstehen sind.

Wie bereits angedeutet, erweist es sich als vorteilhaft, wenn der betrachtete Hygieneartikel, und zwar vorzugsweise einschließlich seiner absorbierenden Bereiche und seiner Chassiskomponenten unzerstört getestet wird, wobei im Hinblick auf das flächenhafte Ausbreiten des Hygieneartikels etwa störende Elastifizierungsmittel entfernt oder deaktiviert werden können.

Die Einleitung der Flüssigkeit in den Hygieneartikel kann in vorteilhafter Weise unter Verwendung einer Testpuppe erfolgen, an die der Hygieneartikel angelegt wird. Dabei kann entsprechend der wunschgemäß zu betrachtenden Tragesituation die Flüssigkeitseinleitung z. B. dann erfolgen, wenn die Testpuppe mit dem angelegten Hygieneartikel in Rückenlage oder in Seitenlage gebracht ist oder auch aufrecht steht oder sitzt. Hierdurch wird nämlich die Flüssigkeitsverteilung innerhalb des Hygieneartikels und seines Absorptionskörpers beeinflusst, was durch die anschließende messtechnische Untersuchung des Hygieneartikels in den zonierten Messarealen quantitativ erfasst werden kann.

In weiterer Ausbildung der Erfindung erweist es sich als vorteilhaft, dass zum Definieren und gegebenenfalls zum körperlichen Abgrenzen der zonierten Messareale eine Schablone verwendet wird. Diese Schablone kann beispielsweise eine Vielzahl von in Querreihen und Längsreihen angeordneter gleich großer oder unterschiedlich großer zonierter Messareale aufweisen, die an sich beliebige geometrische Form aufweisen können, jedoch praktischerweise quadratisch oder rund ausgebildet sind. Die Schablone kann dabei starr oder in bevorzugter Weise etwas biegsam ausgebildet sein. Sie kann beispielsweise eine Markierung aufweisen, und mittels dieser Markierung gegenüber einer Markierung, insbesondere einem geometrischen Mittelpunkt des Hygieneartikels bezogen auf das Chassis oder vorzugsweise bezogen auf den absorbierenden Bereich oder gegenüber einer Randkante oder dergleichen Markierung des Hygieneartikels ausgerichtet und auf dem flächenhaft ausgebreiteten Hygieneartikel platziert werden.

Die erwähnte Schablone weist dabei in vorteilhafter Weise Durchgangsöffnungen auf, die durch Stege voneinander abgegrenzt sind. Die Höhe der Stege kann typischerweise im Bereich einiger Millimeter bis zweckmäßigerweise 20 mm liegen. Ihre Breite wird typischerweise einige wenige Millimeter, insbesondere 1 bis 8 mm, betragen.

Die Abmessung der Messareale, etwa Länge bw. Breite, beträgt in bevorzugter Weise in einer Richtung 10 - 100 mm, insbesondere 10 - 80 mm, insbesondere 20 - 60 mm, insbesondere 30 - 50 mm und in einer anderen Richtung 10 - 100 mm, insbesondere 10 -80 mm, insbesondere 20 - 60 mm, insbesondere 30 - 50 mm.

Der Anteil der durch die Stege überdeckten Fläche des Hygieneartikels bezogen auf die Gesamtfläche der Messareale liegt vorzugsweise bei unter 20 %, insbesondere bei unter 15 % und weiter insbesondere bei unter 10 %.

Die Messwertnahme in einem jeweiligen zonierten Messareal kann in an sich beliebiger Weise erfolgen. Es erweist sich als vorteilhaft, wenn die Messwertnahme durch Gewichtsdifferenzbestimmung, durch Messung einer elektrischen Eigenschaft, durch insbesondere spektroskopische Messung einer optischen Eigenschaft, insbesondere durch Messung mittels Mikrowellen, oder durch Messung der Wärmeleitfähigkeit ausgeführt wird. Auf diese Weise lässt sich jeweils ein Maß für die Menge der in einem jeweiligen Messareal des Hygieneartikels aufgenommenen Flüssigkeit bestimmen. Die vorstehend erwähnte Messwertnahme durch Gewichtsdifferenzbestimmung erfasst beispielsweise auch die Betrachtung des Rücknässungsverhaltens, indem von einem betreffenden Messareal nicht dauerhaft gebundene Flüssigkeit auf ein Transfermittel übertragen und die Menge der übertragenen Flüssigkeit durch an sich beliebige Messwertnahme, insbesondere durch Gewichtsdifferenzmessungen an dem Transfermittel bestimmt wird.

Hierbei erweist es sich auch als vorteilhaft, wenn der Messwert dadurch bestimmt wird, dass auf ein betreffendes Messareal ein Transfermittel für Flüssigkeit aufgebracht wird und das Transfermittel und das betreffende Messareal mit Druck beaufschlagt werden, so dass Flüssigkeit aus dem Messareal in das Transfermittel gelangen kann, und der Messwert dann an dem Transfermittel genommen wird. Bei den erwähnten Transfermitteln kann es sich in vorteilhafter Weise um Filterpapiere handeln.

Nach einer weiteren Ausführungsform der Erfindung wäre es denkbar und vorteilhaft, dass ein flächenhaft erstrecktes plattenförmiges und Flüssigkeit aufnehmendes poröses Transfermittel verwendet wird, welches zumindest über einen Bereich der flächenhaften Erstreckung des ausgebreiteten Hygieneartikels aufgebracht oder aufgelegt wird, wobei solchenfalls das Transfermittel seinerseits vorzugsweise zoniert ausgebildet ist. Hierbei tritt dann in dem Hygieneartikel gespeicherte Flüssigkeit in das flächenhaft erstreckte plattenförmige poröse Transfermittel über. Unter Verwendung des Transfermittels wird quasi ein Spiegelbild oder Abdruck der Flüssigkeitsverteilung in dem Hygieneartikel gewonnen und auf das Transfermittel übertragen. Bereiche bzw. zonierte Messareale des Hygieneartikels werden dadurch auf das flächenhaft erstreckte poröse Transfermittel abgebildet. Bereiche, in denen mehr (nicht dauerhaft gebundene) Flüssigkeit aufgenommen ist, geben entsprechend mehr Flüssigkeit an das Transfermittel ab als Bereiche, in denen weniger nicht dauerhaft gebundene Flüssigkeit zur Verfügung steht. Wann immer ein Transfermittel verwendet wird, werden die an dem Transfermittel genommenen Messwerte auch eine Information über das Rücknässungsverhalten des Hygieneartikels bereitstellen.

Es wäre grundsätzlich denkbar, dass das flächenhaft erstreckte plattenförmige und Flüssigkeit aufnehmende poröse Transfermittel über seine flächenhafte Erstreckung homogen, d.h. a priori nicht zoniert, ausgebildet ist; solchenfalls erweist es sich als vorteilhaft, wenn im Wesentlichen unmittelbar, d.h. ohne wesentliche Verzögerung, an die spiegelbildliche Übertragung der Flüssigkeitsverteilung in dem Hygieneartikel auf das Transfermittel anschließend die Messwertnahme erfolgen würde, so dass eine Aussage über die Flüssigkeitsverteilung in dem Hygieneartikel, also in Bezug auf über den Hygieneartikel zumindest virtuell aufgebrachte zonierte Messareale getroffen werden kann, ohne dass weitere Veränderungen durch Flüssigkeitsleitung in dem Transfermittel auftreten.

In Weiterbildung des flächenhaft erstreckten plattenförmigen porösen Transfermittels erweist es sich als vorteilhaft, wenn dieses Transfermittel selbst zoniert ausgebildet ist, indem es eine Flüssigkeitsaufnahme und/oder Flüssigkeitsleitung verhindernde Trennstege ohne Porosität oder eine Flüssigkeitsaufnahme und/oder Flüssigkeitsleitung verhindernde Einschnitte in einer Z-Richtung aufweist. Wenn die dreidimensionale poröse Struktur des plattenförmigen Transfermittels nichtporöse Trennstege aufweist, so werden voneinander getrennte dreidimensionale Messareale gebildet, zwischen denen eine Flüssigkeitsleitung nicht möglich ist. Es wird so in vorteilhafter Weise verhindert, dass sich die spiegelbildlich übertragene Abbildung der Flüssigkeitsspeicherung von dem Hygieneartikel auf das Transfermittel wieder verändert, indem es zu kapillaren Effekten innerhalb des Transfermittels kommt. Dasselbe kann durch in Z-Richtung erstreckte Einschnitte oder Trennschlitze in dem Transfermittel realisiert werden.

Insbesondere wenn Aussagen über das Rücknässungsverhalten von Hygieneartikeln gemacht werden, erweist es sich als vorteilhaft, wenn das flächenhaft erstreckte plattenförmige und Flüssigkeit aufnehmende poröse Transfermittel und der flächenhaft ausgebreitete Hygieneartikel vor und/oder während einer Messwertnahme gegeneinander gedrückt werden. Auf diese Weise kann eine Tragesituation simuliert werden. Dieses Gegeneinanderdrücken von plattenförmigem Transfermittel und Hygieneartikel kann dadurch realisiert werden, dass das Transfermittel unter vorgegebenem Druck auf den flächenhaft ausgebreiteten Hygieneartikel aufgedrückt wird. Hierbei kann der Hygieneartikel zudem auf einer dreidimensional strukturierten Auflage liegen, um einen über die flächenhafte Erstreckung variierenden Druck vorzusehen. Alternativ könnte auch eine Druckausübung ausgehend von der dem Transfermittel gegenüberliegenden Seite des Hygieneartikels praktiziert werden.

Unabhängig von der Verwendung eines oder mehrerer Transfermittel erweist es sich als vorteilhaft, wenn alle Messareale oder ein Teil der Messareale vor der Messwertnahme mit einem Druck beaufschlagt werden und der Druck für alle oder einen Teil der Messareale entsprechend einer zu simulierenden Tragesituation des Hygieneartikels individuell vorgegeben wird. Somit kann eine Druckbeaufschlagung des Hygieneartikels vor der Messwertnahme dazu eingesetzt werden, möglichst wirklichkeitsnahe Verhältnisse zu schaffen, die wiederum die Flüssigkeitsverteilung innerhalb des Hygieneartikels beeinflussen. Im Ergebnis erhält man so anhand der Messwerte wirklichkeitsnahe Informationen über die Funktion des Hygieneartikels.

Messareale, die im Randbereich des absorbierenden Bereichs des Hygieneartikels liegen, werden bei der Messwertnahme vorzugsweise nicht berücksichtigt, wenn das Messareal zu weniger als etwa 33 % den absorbierenden Bereich überdeckt. Der Flächenanteil nicht gemessener Randbereiche kann in Abhängigkeit von der Kontur und der Produktgröße zwischen etwa 0,5 und etwa 8,0 % an der gesamten absorbierenden Fläche liegen.

Ein wesentlicher Gedanke der vorliegenden Erfindung ist nach alledem darin zu sehen, dass eine Mehrzahl von zonierten Messarealen, die vorzugsweise einen beträchtlichen Teil von insbesondere wenigstens 50% der flächenhaften Erstreckung des absorbierenden Bereichs des Hygieneartikels erfassen, gleichzeitig betrachtet und zur Bestimmung der Feuchtigkeit im weitesten Sinn gleichzeitig oder quasi gleichzeitig messtechnisch untersucht werden. Der Begriff der Gleichzeitigkeit ist dabei so zu verstehen, dass der die Messareale umfassende Hygieneartikel als Ganzes mit Flüssigkeit beaufschlagt wird, die sich dann in dem Hygieneartikel entsprechend dessen Flüssigkeitsaufnahmecharakteristik verteilt und je nach Zusammensetzung der absorbierenden Komponenten des Hygieneartikels (beispielsweise Zellstoff oder superabsorbierende Materialien) mehr oder weniger dauerhaft gespeichert wird. Nach einer vorgegebenen Zeit wird dann eine Mehrzahl von zonierten Messarealen bei dem Hygieneartikel gleichzeitig betrachtet und messtechnisch erfasst, auch wenn die eigentliche Messwertnahme nicht exakt gleichzeitig für alle Messareale, sondern aufeinander folgend durchgeführt wird. Dies wäre beispielsweise der Fall, wenn ein spektroskopischer Sensor bei der Messwertnahme ein Messareal nach dem anderen abtastet oder die Gewichtsdifferenzbestimmung bei zuvor gleichzeitig auf den Hygieneartikel aufgedrückten Transfermitteln aufeinanderfolgend durchgeführt wird.

Gegenstand der vorliegenden Erfindung ist auch eine Vorrichtung zur Durchführung des erfindungsgemäßen Verfahrens, wobei die Vorrichtung eine Einrichtung aufweist zum Bestimmen der Feuchtigkeit in einem jeweiligen zu berücksichtigenden Messareal oder in Bezug auf ein jeweiliges zu berücksichtigendes Messareal, wobei die Einrichtung eine Schablone umfasst, die auf den flächenhaft ausgebreiteten Hygieneartikel auflegbar ist und die Messareale definiert. Es kann sich hierbei beispielsweise um eine Einrichtung handeln, bei der die Messwertnahme unmittelbar bei den zonierten Messarealen erfolgt, indem die Einrichtung in eine Messposition bei dem auf der Vorrichtung flächenhaft ausgebreiteten Hygieneartikel gebracht wird und dann die zonierten Messareale entweder zeitlich aufeinanderfolgend oder gleichzeitig messtechnisch erfasst werden. Beispielsweise könnten die zonierten Messareale zwar aufeinanderfolgend jedoch dennoch im Wesentlichen gleichzeitig im Zuge einer scannenden Messwertnahme ermittelt werden.

In weiterer Ausbildung der Erfindung ist die Vorrichtung zur Durchführung des Verfahrens gekennzeichnet durch eine der Anordnung der zonierten Messareale entsprechende Anordnung von Mitteln zum Ausüben eines Drucks auf die Messareale. Auf diese Weise kann entweder ein Betriebsdruck auf den Hygieneartikel simuliert werden, wie er typischerweise bei verschiedenen Tragesituationen auftritt, oder die Anordnung von druckausübenden Mitteln dient der Befeuchtung von Transfermitteln in Form von Filterpapieren oder dergleichen, um die Rücknässung (rewet, wetback) des Hygieneartikels durch ungebundene Flüssigkeit in den zonierten Messarealen zu erfassen.

Die Mittel zum Ausüben eines Drucks könnten im einfachsten Fall durch eine den Hygieneartikel zumindest bereichsweise überfangende zonierte oder unzonierte Platte gebildet sein, die auf den flächenhaft ausgebreiteten Hygieneartikel aufgelegt, und insbesondere aufgedrückt wird. Vorzugsweise könnte die Platte durchsichtig oder zumindest für Mikrowellen durchlässig ausgebildet sein. Auf diese Weise wäre es sogar möglich, während des Ausübens von Druck eine Messung bei den Messarealen des Hygieneartikels vorzunehmen, insbesondere wenn optische und insbesondere spektroskopische Messverfahren, insbesondere unter Verwendung von Mikrowellen oder Infrarot, zum Einsatz kommen. Hierbei ist es auch möglich, den ausgeübten Druck zu variieren und dann gewissermaßen druckabhängig Flüssigkeitsspeicherprofile anhand der Messwertnahme in den zonierten Messarealen oder in Bezug auf die zonierten Messareale zu gewinnen. Hierbei ist es - wie oben ausgeführt - auch denkbar, dass der Hygieneartikel auf eine vorgegebene dreidimensional strukturierte Oberfläche aufgelegt wird, um unterschiedliche Drücke zu simulieren, oder es wäre denkbar, dass von der gegenüberliegenden Seite der Platte ein insbesondere über die flächenhafte Erstreckung variierender Druck auf den Hygieneartikel ausgeübt wird und entsprechende Mittel hierfür vorgesehen sind.

Weiter erweist es sich als vorteilhaft, wenn die Anordnung von Mitteln zum Ausüben eines Drucks auf die Messareale durch die Schablone hindurchgreift. Solchenfalls wird also die Schablone vorzugsweise auf die körperzugewandte Seite des flächenhaft ausgebreiteten Hygieneartikels aufgelegt. Wenn Transfermittel verwendet werden, so können diese in die Messareale definierenden Öffnungen der Schablone eingelegt werden, und hieran anschließend greifen die Mittel zum Ausüben eines Drucks auf die Messareale durch die Schablone hindurch und belasten die Transfermittel und den Hygieneartikel.

Nach einem weiteren Erfindungsgedanken erweist es sich als vorteilhaft, wenn die Mittel zum Ausüben eines Drucks auf die Messareale so ausgebildet sind, dass je Messareal ein individuell vorgebbarer Druck ausübbar ist. Auf diese Weise lässt sich eine an sich beliebige Trage- oder Belastungssituation des Hygieneartikels simulieren, so dass währenddessen ein Gleichgewicht im Hinblick auf die Flüssigkeitsaufnahme, -verteilung und -speicherung in den absorbierenden Komponenten des Hygieneartikels erreicht werden kann, welcher Gleichgewichtszustand dann der Messung in den zonierten Messarealen zugrunde gelegt wird.

Es erweist sich weiter als vorteilhaft, wenn die Vorrichtung eine eine flächenhafte Unterlage zum Auflegen des Hygieneartikels bildende Basis und eine mechanische Einrichtung umfasst, um die Anordnung von Mitteln zum Ausüben eines Drucks auf die Messareale in eine Arbeitsstellung gegenüber der flächenhaften Unterlage zu bringen. Die mechanische Einrichtung kann dabei portalartig ausgebildet sein, so dass sich ein Portalquerträger, an dem die Mittel zum Ausüben eines Drucks angeordnet sind, oberhalb der flächenhaften Unterlage und damit des Hygieneartikels erstreckt. Alternativ könnte eine Anordnung von Mitteln zum Ausüben eines Drucks auf den Hygieneartikel auch in die Auflage für den Hygieneartikel integriert sein, so dass lediglich von oben ein flächenhaftes Gegendruckmittel erforderlich ist.

Die Anordnung von druckausübenden Mitteln kann eine Mehrzahl von, vorzugsweise individuell steuerbaren und/oder unabhängig voneinander bewegbaren Stempeln umfassen, die gegen die Messareale anlegbar sind.

Insbesondere zur Beurteilung des Rücknässungsverhaltens erweist es sich als vorteilhaft, wenn ein flächenhaft erstrecktes plattenförmiges und Flüssigkeit aufnehmendes poröses Transfermittel vorgesehen ist, welches zumindest über einen Bereich der flächenhaften Erstreckung des ausgebreiteten Hygieneartikels aufbringbar ist, und wenn die Einrichtung zum Bestimmen der Feuchtigkeit zur Messwertnahme an dem flächenhaft erstreckten plattenförmigen Transfermittel ausgebildet ist. Solchenfalls kann wie oben beschrieben ein spiegelbildliches Abbild der Flüssigkeitsspeicherung in dem Hygieneartikel auf das flächenhaft erstreckte poröse Transfermittel übertragen werden, wobei solchenfalls die Messwertnahme an dem Transfermittel stattfindet, wodurch aber gleichwohl eine Aussage über die Flüssigkeitsmenge in einem bestimmten Bereich des Hygieneartikels, also in einem zonierten Messareal des Hygieneartikels, gewonnen werden kann. Auch in diesem Fall erweist es sich als vorteilhaft, wenn eine Einrichtung zum Gegeneinanderdrücken des flächenhaft ausgebreiteten Hygieneartikels und des darauf aufgebrachten flächenhaft erstreckten plattenförmigen und Flüssigkeit aufnehmenden porösen Transfermittels vorgesehen ist. Diese Einrichtung kann Mittel zum Ausüben eines Drucks umfassen, welche den Hygieneartikel von der dem Transfermittel gegenüberliegenden Seite beaufschlagen.

Weitere Merkmale, Einzelheiten und Vorteile der Erfindung ergeben sich aus den beigefügten Patentansprüchen und aus der zeichnerischen Darstellung und Beschreibung des erfindungsgemäßen Verfahrens. In der Zeichnung zeigt:
- Figur 1: eine schematische Draufsicht auf einen absorbierenden Hygieneartikel mit angedeuteten zonierten Messarealen;
- Figuren 2a-e: verschiedene Ansichten einer Messvorrichtung;
- Figur 3: eine schematische Draufsicht auf einen absorbierenden Hygieneartikel mit vermessenen Messarealen und nicht-vermessenen Messarealen im Randbereich
- Figur 4a: eine perspektivische Ansicht eines plattenförmigen porösen Transfermittels; und
- Figur 4b: eine Schnittansicht durch das Transfermittel in einer Messanordnung.

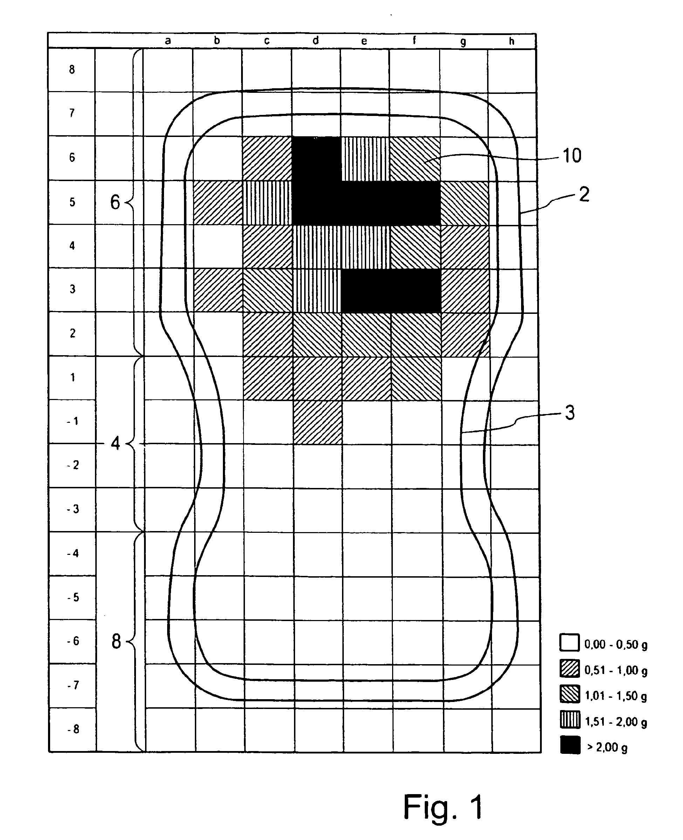

Figur 1 zeigt in schematischer Draufsicht einen absorbierenden Hygieneartikel in Form einer Inkontinenzvorlage 2 mit einer Breite von ungefähr 300 mm und einer Länge von ungefähr 600 mm. Die Inkontinenzvorlage 2 umfasst einen absorbierenden Bereich 3. Dabei kommt der tailliert angedeutete Schrittbereich 4 zwischen den Beinen des Benutzers zu liegen. Der Rückenbereich 6 erstreckt sich nach hinten über das Gesäß des Benutzers und der Vorderbereich 8 erstreckt sich in Richtung auf die Bauchseite des Benutzers. Die Inkontinenzvorlage 2 kann beispielsweise mittels einer Netzhose am Körper des Benutzers gehalten werden. Selbstverständlich sind auch Hygieneartikel von der Erfindung erfasst, die mittels Verschlusselementen auf sich selbst geschlossen werden und so am Körper des Benutzers getragen werden können.

In Figur 1 ist weiter eine Mehrzahl von zonierten Messarealen 10 angedeutet, die beispielhaft quadratische Abmessung mit einer Seitenlänge von ca. 40 mm haben.

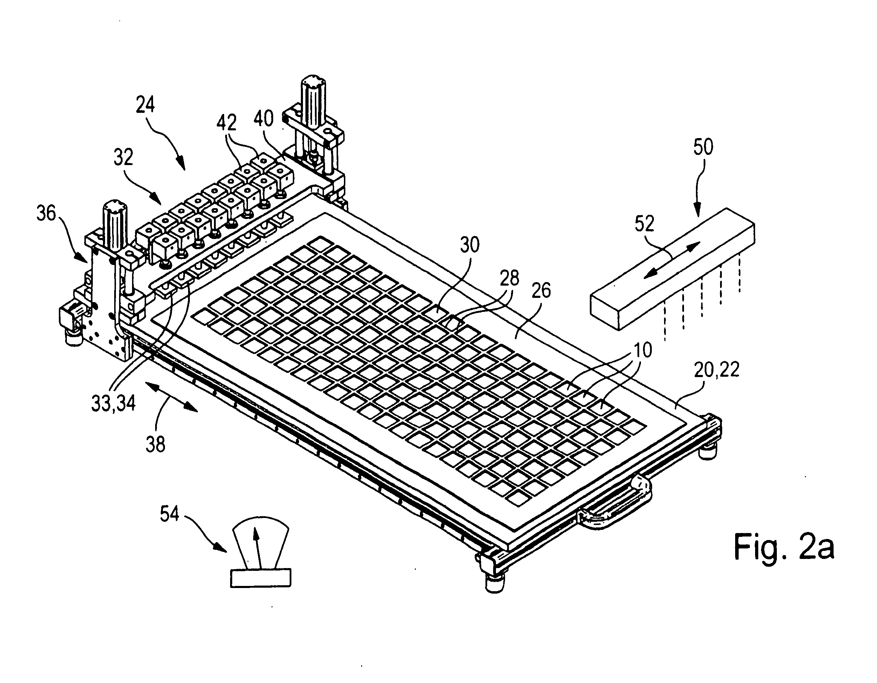

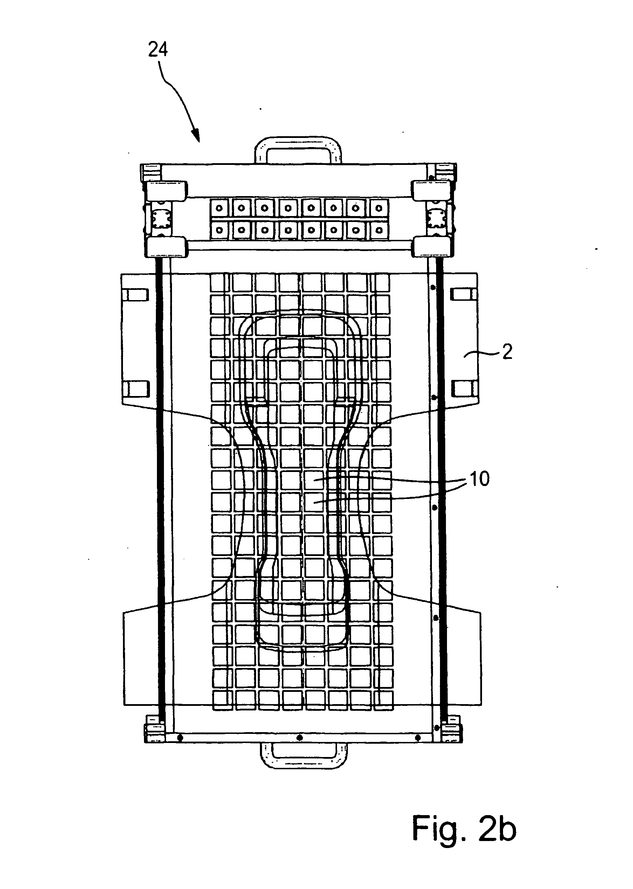

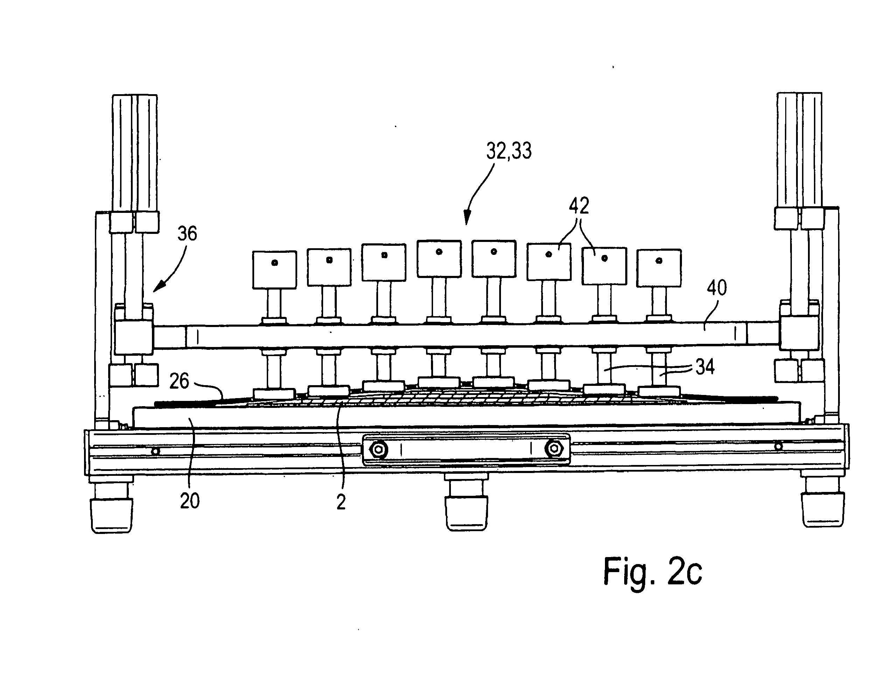

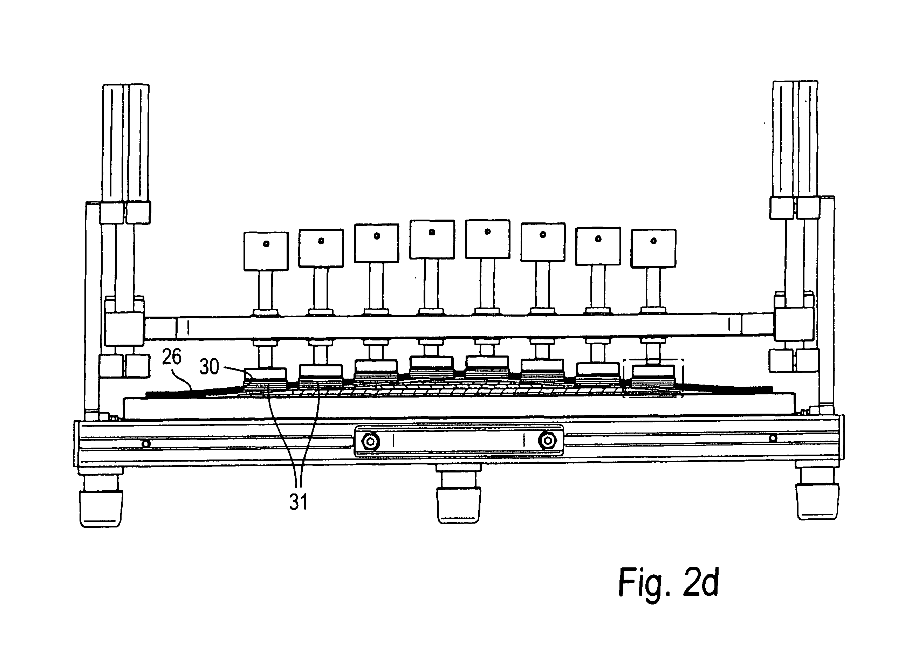

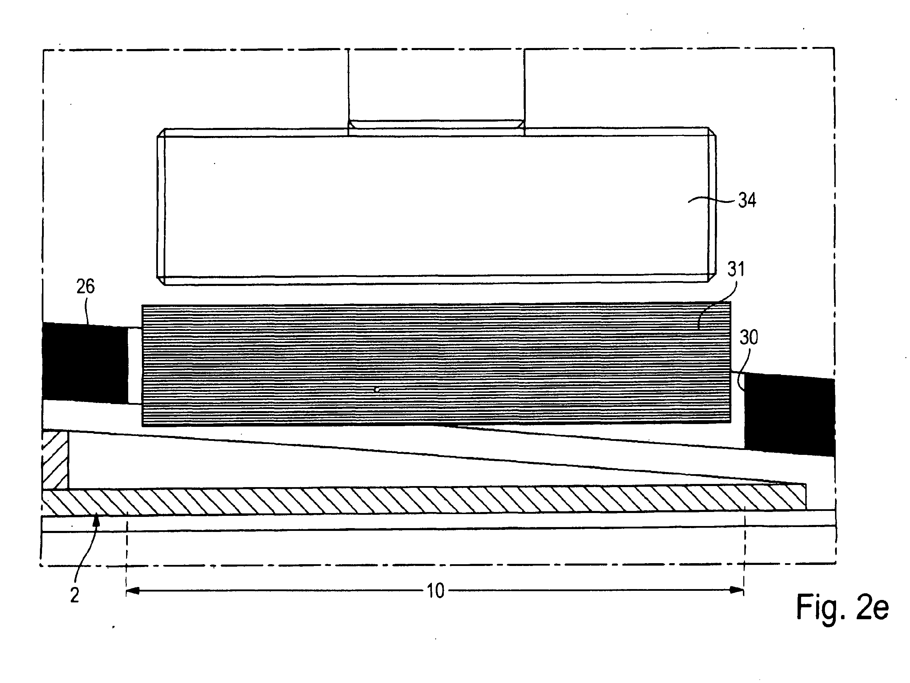

Erfindungsgemäß wird zu jedem Messareal 10 oder zu einer Mehrzahl von Messarealen 10 in einem interessierenden Bereich des Hygieneartikels ein Messwert bestimmt, der in einem Zusammenhang zu der in dem betreffenden Messareal gespeicherten Flüssigkeitsmenge steht. Hierfür wird der Hygieneartikel in einem vorzugsweise standardisierten Verfahren mit einer bestimmten Flüssigkeitsmenge beaufschlagt. Dies kann beispielhaft im flächenhaft ausgebreiteten Zustand des Hygieneartikels wie in Figur 1 angedeutet geschehen, oder der Hygieneartikel wird in bestimmungsgemäßer Weise an einen Benutzer angelegt und mit Flüssigkeit beaufschlagt, oder er wird an eine dem menschlichen Körper nachempfundene Testvorrichtung in Form einer Puppe angelegt und im angelegten Zustand, gegebenenfalls mit zusätzlicher Simulation von Belastungen, im stehenden, liegenden oder sitzenden Zustand mit Flüssigkeit beaufschlagt. Vorzugsweise werden dabei mehrere, insbesondere vier Miktionen, einer bestimmten Flüssigkeitsmenge, beispielsweise jeweils 200 ml, in einem vorgegebenen Zeitabstand von insbesondere 5 Minuten in den Hygieneartikel eingebracht. Nach einem weiteren Zeitintervall von beispielsweise 5 Minuten wird der Hygieneartikel von dem Benutzer oder der Testvorrichtung (Puppe) abgenommen und flächenhaft ausgebreitet, beispielsweise auf einer eine flächenhafte Unterlage 20 bildenden Basis 22 einer in Figur 2 dargestellten und insgesamt mit dem Bezugszeichen 24 bezeichneten Messvorrichtung. Zum Definieren einer Vielzahl von zonierten Messarealen 10 wird auf den in Figur 2 nicht dargestellten flächenhaft ausgebreiteten Hygieneartikel eine Schablone 26 aufgelegt. Die Schablone umfasst eine Vielzahl von durch Stege 28 begrenzte Durchgangsöffnungen 30, welche die Vielzahl von Messarealen 10 begrenzen bzw. definieren. Es wird aber ausdrücklich darauf hingewiesen, dass zum Definieren der Vielzahl von zonierten Messarealen 10 nicht zwingend eine Schablone verwendet werden muss.

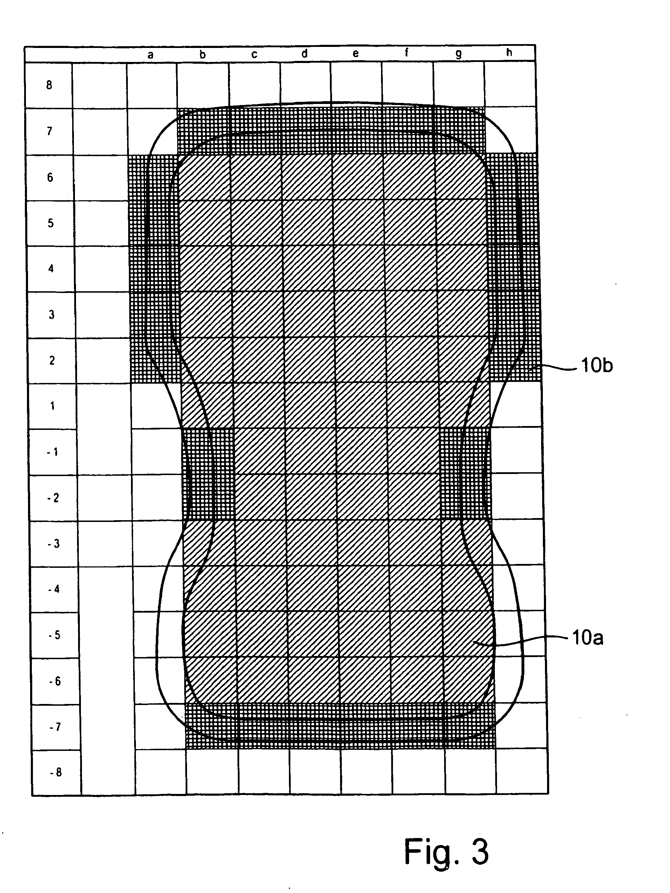

Durch diese Öffnungen 30 hindurch kann für ein jeweiliges Messareal 10 ein Messwert genommen werden, der im Zusammenhang mit der Feuchtigkeit bzw. mit der in dem Hygieneartikel aufgenommenen Flüssigkeit steht. Figur 3 zeigt die Messareale 10a, für die ein Messwert genommen wird, und die Messareale im Randbereich 10b, die zu weniger als etwa 33 % den absorbierenden Bereich überdecken, und für die vorzugsweise kein Messwert genommen oder bei der Auswertung berücksichtigt wird.

Beispielsweise kann als Maß für das Rücknässungsverhalten des Hygieneartikels die aus dem Hygieneartikel entnehmbare, also rücknässende Flüssigkeitsmenge ermittelt werden. Hierfür kann in ein jeweiliges Messareal 10 eine Anordnung von Transfermitteln 31, beispielsweise in Form eines zehnlagigen Stapels von Filterpapieren (Typ No. 3 Whatman) eingelegt werden, die dann durch Druck auf die betreffenden Messareale gegen die körperzugewandte Oberseite des eingenässten Hygieneartikels gedrückt werden, wobei die Filterpapiere nur lose gespeicherte Flüssigkeit aufzunehmen vermögen. Dies wird dann durch Gewichtsdifferenzbestimmung zwischen dem trockenen und befeuchteten Filterpapierstapel gemessen (Angabe in Gramm, Messgenauigkeit 0,01 g). Die Schablone 26 stützt oder begrenzt und hält die Anordnung der Transfermittel 31 in Form von Filterpapieren in dem jeweiligen Messareal 10.

Die Vorrichtung 24 umfasst eine arrayartige Anordnung von beispielhaft 8 x 2 druckerzeugenden Mitteln 33 in Form von Stempeln 34, die senkrecht zur Ebene der flächenhaften Unterlage 20 durch die Öffnungen 30 der Schablone 26 hindurch auf den Hygieneartikel absenkbar sind. Die Stempel 34 sind unabhängig voneinander absenkbar. Man erkennt anhand der Figuren 2c und 2d, dass die unabhängig voneinander absenkbaren Stempel 34 sich so der Topologie des Absorptionskörpers anpassen können, die je nach Flüssigkeitsbeladung ortsabhängig stark variiert. Superabsorbierende Polymermaterialien können nämlich beträchtliche Flüssigkeitsmengen speichern, indem sie in einen gelierten Zustand übergehen und dabei beträchtlich quellen. Figur 2c zeigt einen Querschnitt durch die Messeinrichtung nach Figuren 2a,b ohne Transfermittel 31 und Figur 2d mit Transfermitteln. Die vorzugsweise, jedoch nicht notwendigerweise vorgesehene Schablone 26 ist in beiden Fällen verwandt. Figur 2e verdeutlicht in vergrößerter Ausschnittsdarstellung die haltende oder zentrierende Wirkung der Schablone 26 auf die Positionierung der Transfermittel 31 in den jeweiligen Messarealen 10, insbesondere im Bereich einer stufenförmigen Absorptionskörpertopologie.

Die Anordnung 32 von druckerzeugenden Mitteln ist durch eine mechanische Einrichtung 36 längsverschieblich in Richtung des Doppelpfeils 38 gehalten und lässt sich so in eine Arbeitsstellung gegenüber den zonierten Messarealen 10 des Hygieneartikels bringen. Die mechanische Einrichtung 36 ist portalartig ausgebildet, und die Anordnung 32 mit den Stempeln 34 ist an einem die flächenhafte Unterlage 20 überfangenden und horizontal verlaufenden Querträger 40 angeordnet. Die Druckkraft, mit der die Stempel 34 durch die Öffnungen 30 der Schablone 26 hindurch den Hygieneartikel beaufschlagen, resultiert im hier beispielhaft dargestellten Fall durch die Gewichtskraft von auf die Stempel 34 aufgebrachten Gewichten 42, sie beträgt hier beispielhaft 35 g/cm². Es versteht sich, dass hier weitergehende technische Ausgestaltungen denkbar sind, etwa dahingehend, dass die einzelnen Mittel 33 bzw. Stempel 34 pneumatisch oder hydraulisch oder in sonstiger Weise kraftbeaufschlagbar sind und/oder bei jedem einzelnen Mittel 33 bzw. Stempel 34 zur Druckbeaufschlagung ein individueller Druck einstellbar ist. Dies eröffnet die Möglichkeit, die Mittel 33 zur Druckbeaufschlagung nicht lediglich zur Befeuchtung der Transfermittel einzusetzen, sondern auch um verschiedene Belastungssituationen bei dem eingenässten Hygieneartikel zu simulieren, bevor Messwerte genommen werden. Bei der in der Figur 2a beispielhaft dargestellten Messvorrichtung 24 werden jeweils zwei Zeilen der zonierten Messeareale 10 unter Verwendung der arrayartigen Anordnung 32 der druckbeaufschlagenden Mittel 33 gleichzeitig belastet. Danach werden zwei weitere Zeilen von zonierten Messarealen und darin gegebenenfalls vorgesehene Transfermittel (Figuren 2d,e) belastet. Es versteht sich, dass auch eine gleichzeitige Belastung aller Messareale oder aller im Zuge der Messung zu berücksichtigenden Messareale erfolgen kann.

Die auf diese Weise befeuchteten Transfermittel 31 werden dann wie erwähnt individuell gewogen und die aufgenommene Flüssigkeitsmenge wird bestimmt und dann den einzelnen zonierten Messarealen 10 zugeordnet, was in Figur 1 durch den Eintrag in die Messareale 10 beispielhaft dargestellt ist. Es kann so das Rücknässungsverhalten oder ein anderer im Zusammenhang mit der Absorption von Flüssigkeit stehender Parameter ermittelt werden, und zwar bei einer Mehrzahl von zonierten Messarealen. Die Durchführung der Messung aller zu berücksichtigenden Messareale erfolgt bei 21 - 23°C Raumtemperatur innerhalb von maximal 30 Minuten, um Verdunstungseinflüsse zu minimieren.

In Figur 2a ist weiter schematisch eine Einrichtung 50 zur Bestimmung der Feuchtigkeit bei einem jeweiligen messtechnisch zu berücksichtigenden Messareal 10 vorgesehen, die beispielhaft in Richtung des Doppelpfeils 52 verlagerbar und in eine Arbeitsstellung oberhalb der flächenhaften Unterlage 20 und der Messareale 10 bringbar ist. Beispielsweise kann es sich hierbei um eine spektroskopisch wirkende Messeinrichtung handeln, welche die Messareale 10 messtechnisch erfassen und hierfür in Richtung des Doppelpfeils 38 quasi scannend über die Messareale 10 bewegt werden kann. Sofern, wie vorausgehend beschrieben, mit feuchtigkeitsaufnehmenden Transfermitteln gearbeitet wird, so kann eine schematisch angedeutete Einrichtung 54 zum Bestimmen der Feuchtigkeit eine Wägefunktion für die Transfermittel umfassen.

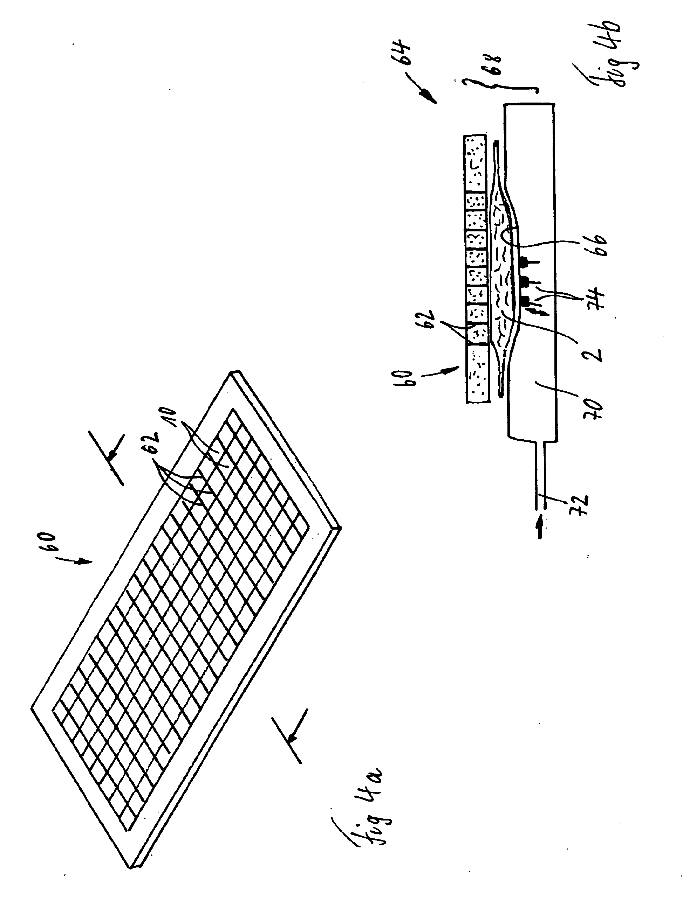

Figur 4a zeigt eine perspektivische Ansicht eines flächenhaft erstreckten plattenförmigen und Flüssigkeit aufnehmenden porösen Transfermittels 60. Dieses Transfermittel 60 ist im bevorzugten Fall zoniert ausgebildet, indem es eine Vielzahl von nichtporösen Stegen 62 aufweist, welche die dreidimensionale poröse Struktur gewissermaßen in Messareale 10 unterteilt, die gegeneinander abgetrennt sind, derart dass zwischen ihnen kein Flüssigkeitstransport innerhalb des Transfermittels 60 stattfinden kann. Alternativ könnten anstelle dieser kreuzförmig zueinander verlaufenden Stege 62 auch Trennschlitze vorgesehen sein, welche sich solchenfalls nicht über die ganze Tiefe der dreidimensional porösen Struktur erstrecken, oder aber es ist dann ein weiterer plattenförmiger Halter für die Messareale, insbesondere in Form einer Plexiglasplatte, vorgesehen.

Figur 4b verdeutlicht eine beispielhaft und nur schematisch dargestellte Messanordnung 64, bei der eine Inkontinenzvorlage 2 auf einer dreidimensional gestalteten Auflage 66 flächenhaft ausgebreitet ist. Darüber ist beispielsweise das in Figur 4a dargestellte plattenförmige poröse Transfermittel 60 aufgelegt, wobei in Figur 4a die Schnittebene angedeutet ist. In diesem Fall bildet das plattenförmige poröse Transfermittel 60 und die Auflage 66 eine Einrichtung 68 zum Gegeneinanderdrücken der Inkontinenzvorlage 2 und des Transfermittels 60. Wenn das Transfermittel 60 gleichmäßig gegen die Auflage 66 gedrückt wird, so stellt sich entsprechend der Formgebung der Auflage 66 und entsprechend der Materialverteilung innerhalb der Inkontinenzvorlage 2 ein vorherbestimmbares Druckprofil ein. Des Weiteren ist in Figur 4b angedeutet, dass der die Auflage 66 bildende Körper 70 beispielsweise expandierbar ausgebildet sein kann, indem beispielsweise über einen Anschluss 72 ein unter Druck stehendes Medium eingebracht wird. Alternativ hierzu könnten in den die Auflage 66 bildenden Körper 70 - wie in der Figur 4b ebenfalls angedeutet ist - in Z-Richtung verstellbare druckausübende Mittel 74 vorgesehen sein. Nachdem die Flüssigkeitsverteilung innerhalb der Inkontinenzvorlage 2 spiegelbildlich auf das Transfermittel 60 übertragen wurde, werden Messwerte bei dem Transfermittel 60 nach einer der eingangs beschriebenen Messmethoden genommen, um eine quantitative Aussage über die Flüssigkeitsverteilung in den berücksichtigten Messarealen zu gewinnen. Bei geeigneter Ausbildung des Transfermittels 60 wäre es sogar denkbar, dass die Messwerte unter Verwendung eines geeigneten Messinstruments unmittelbar bei der in Figur 4b dargestellten Messanordnung 64 genommen werden, indem das Messgerät auf die obere Seite des Transfermittels messtechnisch zugreift.

## Patentansprüche

1. Verfahren zum Bestimmen der Feuchtigkeit bei einem absorbierenden Hygieneartikel (2), insbesondere bei einer Windel, Inkontinenzvorlage oder -windel, Damenbinde, wobei der Hygieneartikel (2) zuvor unter vorzugsweise vorgegebenen Bedingungen mit Flüssigkeit beaufschlagt und danach flächenhaft ausgebreitet wird, **dadurch gekennzeichnet,** dass über die flächenhafte Erstreckung des ausgebreiteten Hygieneartikels (2) eine Mehrzahl zonierter Messareale (10) definiert wird und dass in den jeweiligen Messarealen (10) oder in Bezug auf die jeweiligen Messareale (10) jeweils ein Messwert zum quantitativen Bestimmen der Feuchtigkeit in dem jeweiligen Messareal genommen wird.

2. Verfahren nachAnspruch 1, **dadurch gekennzeichnet, dass** die Messareale (10) wenigstens 50 %, insbesondere wenigstens 60 %, insbesondere wenigstens 70 %, insbesondere wenigstens 80 % und weiter insbesondere wenigstens 90 % der flächenhaften Erstreckung des absorbierenden Bereichs des Hygieneartikels (2) erfassen.

3. Verfahren nach Anspruch 1 oder 2, **dadurch gekennzeichnet, dass** zum Definieren und gegebenenfalls zum körperlichen Abgrenzen der Messareale (10) eine Schablone (26) verwendet wird, die insbesondere Durchgangsöffnungen (30) aufweist, die durch Stege voneinander abgegrenzt sind.

4. Verfahren nach einem der vorstehenden Ansprüche, **dadurch gekennzeichnet, dass** die Messareale (10) eine Abmessung in einer Richtung von 10 - 100 mm, insbesondere von 10 - 80 mm, insbesondere von 20 - 60 mm, insbesondere von 30 - 50 mm und in einer anderen Richtung von 10 - 100 mm, insbesondere von 10 - 80 mm, insbesondere von 20 - 60 mm, insbesondere von 30 - 50 mm haben.

5. Verfahren nach einem der vorstehenden Ansprüche, **dadurch gekennzeichnet, dass** die Messwertnahme durch Gewichtsdifferenzbestimmung, durch Messung einer elektrischen Eigenschaft, durch insbesondere spektroskopische Messung einer optischen Eigenschaft, insbesondere durch Messung mittels Mikrowellen, oder durch Messung der Wärmeleitfähigkeit ausgeführt wird.

6. Verfahren nach einem der vorstehenden Ansprüche, **dadurch gekennzeichnet, dass** der Messwert dadurch bestimmt wird, dass auf ein betreffendes Messareal (10) ein Transfermittel (31) für Flüssigkeit aufgebracht wird und dass das Transfermittel (31) und das betreffende Messareal mit Druck beaufschlagt werden, so dass Flüssigkeit aus dem Messareal in das Transfermittel (31) gelangen kann und dass der Messwert an dem Transfermittel (31) genommen wird.

7. Verfahren nach einem der vorstehenden Ansprüche, **dadurch gekennzeichnet, dass** zumindest über einen Bereich der flächenhaften Erstreckung des ausgebreiteten Hygieneartikels (2) ein flächenhaft erstrecktes plattenförmiges und Flüssigkeit aufnehmendes poröses Transfermittel (60) aufgebracht wird, welches Transfermittel (60) seinerseits vorzugsweise zoniert ausgebildet ist.

8. Verfahren nach Anspruch 7, **dadurch gekennzeichnet, dass** das flächenhaft erstreckte plattenförmige und Flüssigkeit aufnehmende poröse Transfermittel (60) dadurch zoniert ausgebildet ist, dass es eine Flüssigkeitsaufnahme und/oder Flüssigkeitsleitung verhindernde Trennstege (62) ohne Porosität oder eine Flüssigkeitsaufnahme und/oder Flüssigkeitsleitung verhindernde Einschnitte in einer Z-Richtung aufweist.

9. Verfahren nach Anspruch 7 oder 8, **dadurch gekennzeichnet, dass** das flächenhaft erstreckte plattenförmige und Flüssigkeit aufnehmende poröse Transfermittel (60) und der flächenhaft ausgebreitete Hygieneartikel vor und/oder während einer Messwertnahme gegeneinander gedrückt werden.

10. Verfahren nach einem der vorstehenden Ansprüche, **dadurch gekennzeichnet, dass** alle oder ein Teil der Messareale (10) des Hygieneartikels vor der Messwertnahme mit einem Druck beaufschlagt werden und dass der Druck für alle oder einen Teil der Messareale (10) entsprechend einer zu simulierenden Situation vorgegeben wird.

11. Vorrichtung zur Durchführung des Verfahrens nach einem der vorstehenden Ansprüche, umfassend eine Einrichtung (50, 54) zum quantitativen Bestimmen der Feuchtigkeit in einem jeweiligen zu berücksichtigenden Messareal (10) oder in Bezug auf ein jeweiliges zu berücksichtigendes Messareal (10), wobei die Einrichtung eine Schablone (26) umfasst, die auf den flächenhaft ausgebreiteten Hygieneartikel (2) auflegbar ist und die Messareale (10) definiert.

12. Vorrichtung nach Anspruch 11, gekennzeichnet durch eine der Anordnung der zonierten Messareale entsprechende Anordnung (32) von Mitteln (33, 74) zum Ausüben eines Drucks auf die Messareale (10), die insbesondere so ausgebildet sind, dass je Messareal (10) ein individuell vorgebbarer Druck ausübbar ist.

13. Vorrichtung nach Anspruch 12, **dadurch gekennzeichnet, dass** die Anordnung (32) von Mitteln (33) zum Ausüben eines Drucks auf die Messareale (10) durch die Schablone (26) hindurchgreift.

14. Vorrichtung nach Anspruch 12 oder 13 , umfassend eine flächenhafte Unterlage (20) zum Auflegen des Hygieneartikels (2) bildende Basis (22) und eine insbesondere portalartig ausgebildete mechanische Einrichtung (36), um die Anordnung (32) von Mitteln (33) zum Ausüben eines Drucks auf die Messareale (10) in eine Arbeitsstellung gegenüber der flächenhaften Unterlage (20) zu bringen.

15. Vorrichtung nach einem der Ansprüche 11-14, **dadurch gekennzeichnet, dass** die Anordnung (32) von Mitteln (33) zum Ausüben eines Drucks auf die Messareale (10) eine Mehrzahl von, vorzugsweise individuell steuerbaren und/oder unabhängig voneinander bewegbaren Stempeln (34) umfasst, die gegen die Messareale (10) anlegbar sind.

16. Vorrichtung nach einem der Ansprüche 11-15, **dadurch gekennzeichnet, dass** ein flächenhaft erstrecktes plattenförmiges und Flüssigkeit aufnehmendes poröses Transfermittel (60) vorgesehen ist, welches zumindest über einen Bereich der flächenhaften Erstreckung des ausgebreiteten Hygieneartikels (2) aufbringbar ist, und dass die Einrichtung (50, 54) zum Bestimmen der Feuchtigkeit zur Messwertnahme an dem flächenhaft erstreckten plattenförmigen Transfermittel (60) ausgebildet ist.

17. Vorrichtung nach Anspruch 16, gekennzeichnet durch eine Einrichtung (68) zum Gegeneinanderdrücken des flächenhaft ausgebreiteten Hygieneartikels (2) und des darauf aufgebrachten flächenhaft erstreckten plattenförmigen und Flüssigkeit aufnehmenden porösen Transfermittels (60).

18. Vorrichtung nach Anspruch 17, **dadurch gekennzeichnet, dass** die Einrichtung (68) zum Gegeneinanderdrücken des flächenhaft ausgebreiteten Hygieneartikels (2) und des darauf aufgebrachten Transfermittels (60) Mittel (74) zum Ausüben eines Drucks umfassen, welche den Hygieneartikel von der dem Transfermittel (60) gegenüberliegenden Seite beaufschlagen.

## Claims

1. Method for determining the moisture of an absorbent sanitary product (2), in particular, of a diaper, incontinence pad or incontinence diaper, sanitary towel, wherein liquid is previously applied to the sanitary product (2) preferably under predetermined conditions and the sanitary product is subsequently flatly spread-out, **characterized in that** a plurality of zoned measurement areas (10) are defined over the planar extension of the spread-out sanitary product (2) and that one measured value is taken in each measurement area (10) or with respect to each measurement area (10) for quantitatively determining the moisture in the respective measurement area.

2. Method according to any one of the preceding claims, **characterized in that** the measurement areas (10) include at least 50%, in particular at least 60%, in particular at least 70%, in particular at least 80% and moreover, in particular, at least 90% of the planar extension of the absorbent area of the sanitary product (2).

3. Method according to claim 1 or 2, **characterized in that** a template (26) is used for defining and, if necessary, physically delimiting the measurement areas (10), which in particular comprises through-holes (30).

4. Method according to any one of the preceding claims, **characterized in that** the measurement areas (10) have a size in one direction of 10 to 100 mm, in particular 10 to 80 mm, in particular 20 to 60 mm, in particular 30 to 50 mm, and in another direction of 10 to 100 mm, in particular 10 to 80 mm, in particular 20 to 60 mm, in particular 30 to 50 mm.

5. Method according to any one of the preceding claims, **characterized in that** the measurement is performed by determining the weight difference, by measurement of an electrical property, in particular spectroscopic measurement of an optical property, in particular by measurement using microwaves or by measurement of the heat conductivity.

6. Method according to any one of the preceding claims, **characterized in that** the measured value is determined **in that** a transfer means (31) for liquid is applied to a respective measurement area (10) and the transfer means (31) and the respective measurement area are pressurized such that liquid can pass from the measurement area into the transfer means (31), and the measurement is performed on the transfer means (31).

7. Method according to any one of the preceding claims, **characterized in that** a flatly extending plate-shaped and liquid-absorbing porous transfer means (60) is applied to at least part of the planar extension of the spread-out sanitary product (2), wherein the transfer means (60) itself is advantageously designed in zones.

8. Method according to claim 7, **characterized in that** the flatly extending plate-shaped and liquid-absorbing porous transfer means (60) is zoned **in that** it comprises non-porous separating webs (62), which prevent liquid absorption and/or liquid flow, or cuts in a Z direction, which prevent liquid absorption and/or liquid flow.

9. Method according to claim 7 or 8, **characterized in that** the flatly extending plate-shaped and liquid-absorbing porous transfer means (60) and the spread-out planar sanitary product are pressed against each other prior to and/or during a measurement.

10. Method according to any one of the preceding claims, **characterized in that** all or part of the measurement areas (10) of the sanitary product are pressurized prior to measurement, the pressure being predetermined for all or part of the measurement areas (10) in correspondence with a situation to be simulated.

11. Device for performing the method according to any one of the preceding claims, comprising a means (50, 54) for quantitatively determining the moisture in a respective measurement area (10) to be considered or with respect to a respective measurement area (10) to be considered, wherein the means comprises a template (26) that can be applied to the spread-out planar sanitary product (2) and defines the measurement areas (10).

12. Device according to claim 11, **characterized by** a configuration (32) of means (33, 74) for applying a pressure onto the measurement areas (10), which corresponds to the configuration of the zoned measurement areas, which in particular are designed in such a fashion that each measurement area (10) can be loaded with an individually predeterminable pressure.

13. Device according to claim 12, **characterized in that** the configuration (32) of means (33) for exerting pressure onto the measurement areas (10) extends through the template (26).

14. Device according to claims 12 or 13, comprising a base (22) for forming a planar support (20) for supporting the sanitary product (2), and a mechanical device (36) particularly for moving the configuration (32) of means (33) for exerting a pressure onto the measurement areas (10) into a work position with respect to the planar support (20).

15. Device according to any one of the claims 11 to 14, **characterized in that** the configuration (32) of means (33) for exerting pressure onto the measurement areas (10) has a plurality of pistons (34) that can preferably be individually controlled and/or moved independently of each other and can be applied against the measurement areas (10).

16. Device according to any one of the claims 11 to 15, **characterized in that** a flatly extending plate-shaped and liquid-absorbing porous transfer means (60) is provided, which can be applied at least over part of the planar extension of the spread-out sanitary product (2), and that the means (50, 54) for determining the moisture is designed for measurement on the flatly extending plate-shaped transfer means (60).

17. Device according to claim 16, **characterized by** a device (68) for pressing the spread-out planar sanitary product (2) and the flatly extending plate-shaped and liquid-absorbing porous transfer means (60), disposed on the sanitary product, against each other.

18. Device according to claim 17, **characterized in that** the device (68) for pressing the spread-out planar sanitary product (2) and the transfer means (60) disposed thereon comprise means (74) for exerting a pressure for loading the sanitary product from the side opposite to the transfer means (60).

## Revendications

1. Procédé de détermination de l'humidité dans un article hygiénique (2) absorbant, en particulier dans une couche, une protection ou couche pour l'incontinence, serviette hygiénique, dans lequel ledit article hygiénique (2) est alimenté auparavant, dans des conditions de préférence données, en liquide et est ensuite déployé en nappe, **caractérisé par le fait que** l'on définit une pluralité d'aires de mesure zonées (10) sur l'extension en nappe de l'article hygiénique (2) déployé et que l'on détermine dans les aires respectives de mesure (10) ou par rapport aux aires respectives de mesure (10) respectivement une valeur de mesure pour déterminer de manière quantitative l'humidité dans l'aire de mesure (10) respective.

2. Procédé selon la revendication 1, **caractérisé par le fait que** les aires de mesure (10) occupent au moins 50 %, en particulier au moins 60 %, en particulier au moins 70 %, en particulier au moins 80 % et encore en particulier au moins 90 % de l'extension en nappe de la zone absorbante de l'article hygiénique (2).

3. Procédé selon la revendication 1 ou 2, **caractérisé par le fait que**, pour définir et, le cas échéant, pour délimiter physiquement lesdites aires de mesure (10), on utilise un gabarit (26) qui présente en particulier des ouvertures de passage (30) qui sont délimitées les unes des autres par des entretoises.

4. Procédé selon l'une quelconque des revendications précédentes, **caractérisé par le fait que** les aires de mesure (10) présentent une dimension, dans une direction, comprise entre 10 et 100 mm, en particulier entre 10 et 80 mm, en particulier entre 20 et 60 mm, en particulier entre 30 et 50 mm, et, dans une autre direction, comprise entre 10 et 100 mm, en particulier entre 10 et 80 mm, en particulier entre 20 et 60 mm, en particulier entre 30 et 50 mm.

5. Procédé selon l'une quelconque des revendications précédentes, **caractérisé par le fait que** l'obtention de valeur de mesure est réalisée par détermination de différence de poids, par mesure d'une propriété électrique, par mesure en particulier spectroscopique d'une propriété optique, en particulier par mesure au moyen de microondes ou par mesure de la conductivité thermique.

6. Procédé selon l'une quelconque des revendications précédentes, **caractérisé en ce que** la valeur de mesure est déterminée par le fait qu'un moyen de transfert (31) pour liquide est appliqué sur une aire de mesure (10) en question et que ledit moyen de transfert (31) et l'aire de mesure en question sont soumis à une pression de sorte que du liquide puisse passer depuis l'aire de mesure dans le moyen de transfert (31) et que la valeur de mesure est déterminée sur le moyen de transfert (31).

7. Procédé selon l'une quelconque des revendications précédentes, **caractérisé par le fait que** sur au moins une zone de l'extension en nappe de l'article hygiénique (2) déployé est appliqué un moyen de transfert (60) poreux en forme de plaque, étendu en nappe et absorbant du liquide, lequel moyen de transfert (60) est réalisé de son côté, de préférence, de manière zonée.

8. Procédé selon la revendication 7, **caractérisé en ce que** ledit moyen de transfert (60) poreux en forme de plaque, étendu en nappe et absorbant du liquide est réalisé de manière zonée par le fait qu'il présente des entretoises de séparation (62) exemptes de porosité qui évitent une absorption de liquide et/ou une conduction de liquide ou des incisions dans une direction Z qui évitent une absorption de liquide et/ou une conduction de liquide.

9. Procédé selon la revendication 7 ou 8, **caractérisé par le fait que** ledit moyen de transfert (60) poreux en forme de plaque, étendu en nappe et absorbant du liquide ainsi que ledit article hygiénique déployé en nappe sont plaqués l'un contre l'autre avant et/ou durant une détermination de valeur de mesure.

10. Procédé selon l'une quelconque des revendications précédentes, **caractérisé par le fait que** toutes les ou une partie des aires de mesure (10) de l'article hygiénique sont soumises à une pression avant la détermination de valeur de mesure et que la pression est donnée pour toutes les ou une partie des aires de mesure (10) en fonction d'une situation à simuler.

11. Dispositif de mise en oeuvre du procédé selon l'une quelconque des revendications précédentes, comprenant un dispositif (50, 54) de détermination quantitative de l'humidité dans une aire respective de mesure (10) à prendre en considération ou par rapport à une aire respective de mesure (10) à prendre en considération (10), ledit dispositif comprenant un gabarit (26) qui peut être posé sur l'article hygiénique (2) déployé en nappe et qui définit les aires de mesure (10).

12. Dispositif selon la revendication 11, **caractérisé par** un agencement (32) - correspondant à l'agencement des aires de mesure zonées - de moyens (33, 74) destinés à exercer une pression sur les aires de mesure (10), qui sont réalisés en particulier de manière à ce que l'on puisse exercer, par aire de mesure (10), une pression qui peut être donnée individuellement.

13. Dispositif selon la revendication 12, **caractérisé par le fait que** l'agencement (32) de moyens (33) destinés à exercer une pression sur les aires de mesure (10) traverse ledit gabarit (26).

14. Dispositif selon la revendication 12 ou 13, comprenant une base (22) formant un support (20) en nappe pour y poser l'article hygiénique (2) ainsi qu'un dispositif (36) mécanique en particulier de type portique afin d'amener ledit agencement (32) de moyens (33) destinés à exercer une pression sur les aires de mesure (10), dans une position de travail par rapport au support (20) en nappe.

15. Dispositif selon l'une quelconque des revendications 11 à 14, **caractérisé par le fait que** l'agencement (32) de moyens (33) destinés à exercer une pression sur les aires de mesure (10) comprend une pluralité de tampons (34) qui, de préférence, peuvent être commandés individuellement et/ou être déplacés indépendamment les uns des autres et qui peuvent être appliqués contre les aires de mesure (10).

16. Dispositif selon l'une quelconque des revendications 11 à 15, **caractérisé par le fait que** l'on prévoit un moyen de transfert (60) poreux en forme de plaque, étendu en nappe et absorbant du liquide, qui peut être appliqué au moins sur une zone de l'extension en nappe de l'article hygiénique (2) déployé, et que ledit dispositif (50, 54) de détermination de l'humidité est réalisé pour la détermination de valeur de mesure sur le moyen de transfert (60) en forme de plaque étendu en nappe.

17. Dispositif selon la revendication 16, **caractérisé par** un dispositif (68) destiné à plaquer l'un contre l'autre ledit article hygiénique (2) déployé en nappe et ledit moyen de transfert (60) poreux en forme de plaque, étendu en nappe et absorbant du liquide qui est appliqué sur ce premier.

18. Dispositif selon la revendication 17, **caractérisé par le fait que** ledit dispositif (68) destiné à plaquer l'un contre l'autre ledit article hygiénique (2) déployé en nappe et ledit moyen de transfert (60) appliqué sur celui-ci comprend des moyens (74) destinés à exercer une pression qui sollicitent l'article hygiénique depuis la face située en regard du moyen de transfert (60).
